# EUROPEAN PATENT SPECIFICATION

(11) **EP 3 119 454 B1**
(45) Date of publication and mention of the grant of the patent: **16.05.2018**
(21) Application number: 15710535.4
(22) Date of filing: 19.03.2015
(51) Int. Cl.: A61M 5/32, A61M 5/24, A61M 5/34

(54) **DRUG DELIVERY DEVICE WITH CAP INDUCED NEEDLE MOVEMENT**
WIRKSTOFFVERABREICHUNGSVORRICHTUNG MIT KAPPEINDUZIERTER NADELBEWEGUNG
DISPOSITIF D'INJECTION DE MÉDICAMENT AVEC MOUVEMENT D'AIGUILLE INDUIT PAR CAPUCHON

(30) Priority: 21.03.2014 EP 14161070
(43) Date of publication of application: 25.01.2017
(73) Proprietor: Novo Nordisk A/S, 2880 Bagsværd (DK)
(72) Inventor: SØRENSEN, Morten, 2880 Bagsværd (DK); ENGGAARD, Christian Peter, 2880 Bagsværd (DK)
(86) International application number: PCT/EP2015/055826
(87) International publication number: WO 2015/140262

(56) References cited:
- EP-A1- 2 018 885
- WO-A1-2013/053949
- WO-A2-2011/051366

## Description

### FIELD OF THE INVENTION

The present invention relates to medical devices adapted to expel drug from a reservoir through a needle.

### BACKGROUND OF THE INVENTION

Drug delivery devices, such as e.g. injection pens, are widely used for self-administration of liquid drugs by people in need of therapeutic treatment. Many injection devices are capable of repeated setting and injection of either a fixed or a variable volume of drug by operation of respective dose setting and injection mechanisms in the device. Some injection devices are adapted to be loaded with a prefilled drug reservoir containing a volume of drug which is sufficient to provide for a number of injectable doses. When the reservoir is empty, the user replaces it with a new one and the injection device can thus be used again and again. Other injection devices are prefilled when delivered to the user and can only be used until the drug reservoir has been emptied. The various injection devices typically expel the drug by advancing a rubber piston in the reservoir using a motion controlled piston rod.
Conventional injection pens require use of an injection needle to penetrate the skin and administer the drug. Such an injection needle may form part of a needle assembly comprising a needle hub to which the injection needle is adhered, and a collar with means for attachment to a needle receiving part of the injection pen. The injection needle is retained by the needle hub in such a way that a portion of the injection needle protrudes in one axial direction from the needle hub, and another portion of the injection needle protrudes in the other axial direction from the needle hub. This provides for a so-called back needle dedicated to establish fluid communication with an interior of the drug reservoir and a so-called front needle dedicated to establish fluid communication with the human tissue.

A prior art pre-assembled hand-held medical arrangement for transferring substance between containers, one of which is a drug delivery syringe, is given in WO 2013/053949 A1. This medical arrangement has a cover coupled with fluid connection unit and causing relative converging motion between one container and fluid connection unit which ensures that purity of the substances is maintained throughout the mixing procedure. For safety reasons, especially in view of a potential wear of the injection needle and a risk of bacterial contamination, it is recommended that injections pens are used once and then discarded. Nevertheless, many people choose to re-use the injection needles, and some even use the injection needles several times. If the back needle resides in the drug reservoir during storage of the injection device an unspecified volume of the drug may leak out, either due to dripping or evaporation. Leakage of drug may lead to a smaller than intended dose being delivered during the following administration, either because the piston as a result has moved distally, leading to an air gap between the piston and the piston washer, or because the leaked drug has been substituted with an air bubble inside the cartridge. Furthermore, temperature variations during storage of the injection device may enhance these effects due to related changes in the density of the drug. Alternatively, or additionally, the drug may dry out and crystallise in the front needle tip. Capillary effects in the needle will continuously replenish the dried out drug, and eventually the crystallisation may clog the injection needle completely and prevent further injections.

Normally, the user is instructed to perform an air shot prior to each injection in order to eliminate an air gap and to expel air bubbles from the drug reservoir as well as crystallised drug from the injection needle. However, if, for some reason, the air shot is not performed, e.g. due to negligence from the user, the user will not be aware of the risks associated with the upcoming injection. It is thus desirable to provide a drug delivery device which does not present such risks to a user, even if the user leaves a needle element residing in the drug reservoir for a prolonged period of time.

In order to reduce post-dispensing droplet generation WO 2011/051366 (Sanofi-Aventis Deutschland GmbH) suggests to incorporate a compression spring distally of a cartridge in an injection pen for automatic return of the cartridge and an associated drive mechanism to a pre-dispensing position when a thrust provided by the user for administration of a dose of drug is reduced below a predefined threshold. This should provide for a self-actuated disconnection of piercing element and cartridge as soon as a dose dispensing procedure has terminated.

However, apart from the addition of a cost-raising metal component dedicated to effect this return the solution entails a complex type of device where the drive mechanism during the administration procedure firstly has to displace the entire cartridge relative to the housing, and subsequently displace the piston relative to the cartridge while the cartridge is fixed with respect to the housing. In precision delivery devices the movement of the piston rod must be closely correlated with the movement of the piston relative to the cartridge wall to ensure that the correct dose is expelled. For such delivery devices it is preferable that all advancing movements of the piston rod are converted directly to advancements of the piston in the cartridge because dose delivery is based on a well-defined displacement of the piston rod relative to the housing. It is thus undesirable to additionally take account of a part of the piston rod movement being used to advance the cartridge itself.

### SUMMARY OF THE INVENTION

It is an object of the invention to eliminate or reduce at least one drawback of the prior art, or to provide a useful alternative to prior art solutions.

In particular, it is an object of the invention to provide a drug delivery device which does not present a risk of leakage or injection needle clogging even if an injection needle is not removed therefrom between injections.

It is a further object of the invention to provide such a drug delivery device which can operate also without requiring a displacement of the drug reservoir relative to the device housing.

It is an even further object of the invention to provide such a drug delivery device which is cost-effective and easy to operate, and which requires no additional user performed operation steps compared with conventional devices of similar type.

In the disclosure of the present invention, aspects and embodiments will be described which will address one or more of the above objects and/or which will address objects apparent from the following text.

In a first aspect the invention provides a drug delivery device as specified in claim 1.

Accordingly, a drug delivery device is provided comprising a drug delivery unit which comprises a) a housing extending along a general axis, b) a variable volume reservoir, and c) a needle support for receiving a needle assembly, the needle support and the variable volume reservoir being capable of relative axial motion between a first relative position and a second relative position, where relative axial motion from the first relative position to the second relative position involves an axially diverging relative motion between the needle support and the variable volume reservoir, and relative axial motion from the second relative position to the first relative position involves an axially converging relative motion between the needle support and the variable volume reservoir.

Specifically, relative axial motion from the first relative position to the second relative position may consist exclusively of an axially diverging relative motion between the needle support and the variable volume reservoir, and relative axial motion from the second relative position to the first relative position may consist exclusively of an axially converging relative motion between the needle support and the variable volume reservoir.

The drug delivery device further comprises a cap removably mountable onto the drug delivery unit to cover at least a portion of the needle support, and a coupling mechanism configured to bring the needle support and the variable volume reservoir from the first relative position to the second relative position during mounting of the cap onto the drug delivery unit. This ensures that when the cap is mounted on the drug delivery unit the needle support and the variable volume reservoir are spaced apart such that if an injection needle is left on the needle support the back needle will not be able to reside in the reservoir interior and thereby risk becoming clogged. Furthermore, the interruption of the fluid connection between the injection needle and the reservoir interior minimises the risk of drug leaking out of, and air entering into, the variable volume reservoir during storage.

Conventional use of a drug delivery device which includes a drug delivery unit and a cap involves mounting the cap onto the drug delivery unit between dose administrations to protect specific parts of the drug delivery unit, such as the needle support and the drug containing reservoir. The present invention thus provides for an automatic separation of the needle support and the variable volume reservoir without introducing an additional operation step for the user.

The cap is completely mounted on the drug delivery unit when an interface portion of the cap is received and releasably retained by a dedicated cap receiving portion of the drug delivery unit, and it is understood that the coupling mechanism is configured to bring the needle support and the variable volume reservoir to the second relative position during a relative motion between the cap and the drug delivery unit that eventually will lead to the interface portion of the cap being received by the cap receiving portion. The second relative position may be reached, though, before the interface portion of the cap is actually received by the cap receiving portion.

In particular, the coupling mechanism may be configured to convert a relative axial movement between the cap and the housing to an opposite relative axial movement between the needle support and the variable volume reservoir. The relative axial movement between the cap and the housing may be purely axial or merely include an axial component, such as e.g. in the case of a helical, or partially helical, relative movement.

The cap may comprise a longitudinally extending circumferential side wall and a transversal end wall closing one end of the cap such that the needle support is entirely encapsulated by the cap and a portion of the drug delivery unit when the cap is completely mounted on the drug delivery unit. An attached needle assembly will thereby be inaccessible to a user for example when the drug delivery device is stored, with the cap retained by the cap receiving portion, preventing accidental needle stick injuries.

The needle support may be a non-detachable part of the drug delivery unit. In particular, the first relative position and/or the second relative position of the needle support and the variable volume reservoir may be predetermined in the sense that the drug delivery device may be constructed to allow movement of one or both of the needle support and the variable volume reservoir from one predefined relative position to another predefined relative position. This provides a self-contained drug delivery unit having no loose parts, and a drug delivery device wherein each cap mounting brings the needle support to a well-defined position relative to the variable volume reservoir, enabling a guaranteed retraction of an attached needle assembly from the interior of the variable volume reservoir.

The coupling mechanism may further be configured to bring the needle support and the variable volume reservoir from the second relative position to the first relative position during dismounting of the cap from the drug delivery unit. This enables a fully automatic needle retraction from, and needle insertion into, the reservoir interior, carried out by the user's conventional manipulation of the cap without requiring any specific attention thereto. The automatic movement of the needle support and the variable volume reservoir from the second relative position to the first relative position at the time of cap removal also ensures that any excessive pressure in the variable volume reservoir is equalised before the injection needle resides in the user.

Alternatively, the needle support and the variable volume reservoir may be brought from the second relative position to the first relative position by the user manually pushing them towards one another after dismounting of the cap from the drug delivery unit.

The variable volume reservoir may be axially fixed with respect to the housing, and the coupling mechanism may be configured to displace the needle support distally in response to a converging relative axial motion between the cap and the housing during mounting of the cap onto the drug delivery unit. The coupling mechanism may additionally be configured to displace the needle support proximally in response to a diverging relative axial motion between the cap and the housing during dismounting of the cap from the drug delivery unit

Alternatively, the needle support may be axially fixed with respect to the housing, and the coupling mechanism may be configured to displace the variable volume reservoir proximally in response to a converging relative axial motion between the cap and the housing during mounting of the cap onto the drug delivery unit. For example, the cap may be adapted to interface with the variable volume reservoir and push the variable volume reservoir proximally during the mounting of the cap onto the drug delivery unit.

Alternatively, both the needle support and the variable volume reservoir are movable with respect to the housing, and the coupling mechanism may be configured to move them both during mounting of the cap onto the drug delivery unit.

The needle support may be configured to releasably retain the needle assembly to thereby allow use of the drug delivery device with a plurality of injection needles. Particularly, the needle support may comprise a needle mount having a thread and/or a bayonet track for reception of a mating structure in or on the needle assembly. Alternatively, the needle support may be configured to non-releasably retain the needle assembly.

The variable volume reservoir may comprise a reservoir interior and a penetrable self-sealing septum, and the drug delivery device may further comprise a needle assembly attached to the needle support, the needle assembly comprising a hollow access structure configured to penetrate the self-sealing septum. In that case, in the first relative position of the needle support and the variable volume reservoir the hollow access structure is fluidly connected with the reservoir interior through the self-sealing septum, and in the second relative position of the needle support and the variable volume reservoir the hollow access structure is fluidly disconnected from the reservoir interior.

The coupling mechanism may comprise a first interface structure, a second interface structure, and a third interface structure, where each of the first interface structure, the second interface structure, and the third interface structure is configured for interaction with at least one of the other of the first interface structure, the second interface structure, and the third interface structure during at least a part of the mounting of the cap onto, and the dismounting of the cap from, the drug delivery unit. Particularly, the first interface structure may be arranged in or on the cap, and the second interface structure may be arranged in or on the needle support.

In some embodiments of the invention the needle support comprises a first needle support portion adapted to hold the needle assembly and a second needle support portion adapted to receive the first interface structure. The first needle support portion and the second needle support portion extend along the general axis and are arranged eccentrically. This type of needle support is particularly useful with a dual axis delivery device.

The second needle support portion may comprise the second interface structure, and the third interface structure may be axially fixed with respect to the housing and may comprise an exterior portion configured for threaded engagement with the second interface structure, and an interior portion configured for threaded engagement with the first interface structure, such that an axial movement of the first interface structure relative to the third interface structure causes an opposite axial movement of the second needle support portion relative to the third interface structure.

In some embodiments of the invention the third interface structure is a nut element rotatably accommodated in the second needle support portion and comprising an exterior thread, and the second interface structure comprises a protruding structure configured to engage with the exterior thread. The protruding structure is arranged on an interior surface of the second needle support portion. Furthermore, the first interface structure comprises a rod being translationally and rotationally fixed to the transversal end wall of the cap and extending longitudinally therefrom. One of the rod and the nut element comprises a non-self-locking thread, and the other of the rod and the nut element comprises a protruding structure configured to engage with the non-self-locking thread. A relative axial motion between the rod and the nut element will thus cause a rotation of the nut element with respect to the needle support, which rotation due to the threaded engagement between the nut element and the second needle support structure will cause a relative axial motion between the needle support and the nut element. The direction of the thread is chosen to provide a distal motion of the nut element in response to a proximal motion of the rod.

The drug delivery device may further comprise a reservoir support for axially retaining the variable volume reservoir with respect to the housing. The reservoir support may itself be configured for axial and rotational fixation with respect to the housing.

The needle support and the reservoir support may be capable of undergoing relative helical motion during mounting of the cap onto the drug delivery unit. Further, the mounting of the cap onto the drug delivery unit may involve a relative angular displacement between the cap and the reservoir support, and the first interface structure and the second interface structure may be configured to rotationally fix the needle support with respect to the cap.

The drug delivery device may further comprise a fourth interface structure arranged in the cap, the third interface structure may be arranged on the reservoir support, and the third interface structure and the fourth interface structure may be configured to define the relative angular displacement between the cap and the reservoir support.

One of the first interface structure and the second interface structure may comprise an axial groove, and the other of the first interface structure and the second interface structure may comprise a protrusion configured for sliding reception in the axial groove. In addition, one of the third interface structure and the fourth interface structure may comprises an at least partly helical groove, and the other of the third interface structure and the fourth interface structure may comprise a protrusion configured for sliding reception in the at least partly helical groove.

The needle support may further comprise a leg member comprising skewed surfaces configured for sliding interaction with skewed surfaces on the reservoir support such that an induced relative rotational motion between the needle support and the reservoir support will result in an additional relative axial motion between the two.

In another aspect of the invention, a drug delivery device is provided comprising a drug delivery unit which comprises a) a housing extending along a general axis, b) a reservoir support for holding a variable volume reservoir, and c) a needle support for receiving a needle assembly, the needle support and the reservoir support being capable of diverging relative axial motion from a first relative position to a second relative position and converging relative axial motion from the second relative position to the first relative position, a cap removably mountable onto the drug delivery unit to cover at least a portion of the needle support, and a coupling mechanism configured to bring the needle support and the reservoir support from the first relative position to the second relative position in response to the cap being mounted onto the drug delivery unit.

The reservoir support may non-releasably retain a variable volume reservoir, such as e.g. a cartridge having a cylindrical wall closed at an outlet end by a self-sealing penetrable septum and sealed at a distance from the outlet end by a slidable piston, or it may be adapted to allow a held variable volume reservoir to be exchanged. The variable volume reservoir may be prefilled and/or fillable through the septum.

A drug delivery device according to this aspect of the invention provides for an automatic separation of the needle support and the reservoir support during mounting of the cap onto the drug delivery unit, whereby it can be ensured that if a needle assembly is attached to the needle support fluid communication with an interior of a reservoir held in the reservoir support is ended once the drug delivery device is in a storage state where the cap is attached to the drug delivery unit.

The needle support may be arranged non-detachably with respect to the reservoir support to reduce the risk of component misplacement and/or erroneous handling by a user. In any case the needle support and the reservoir support may be capable of a predetermined diverging relative axial motion from the first relative position to the second relative position and a predetermined converging relative axial motion from the second relative position to the first relative position, i.e. the extent of the relative axial motion between the first relative position and the second relative position is defined by the device construction.

In a further aspect of the invention a fluid dispenser is provided comprising A) a dispensing unit comprising a1) a reservoir support for holding a fluid containing reservoir, and a2) a conveyor support for holding a fluid conveying structure of the type having an access structure (such as e.g. a needle or a hollow spike) for establishing fluid communication to an interior of the fluid containing reservoir, and a delivery structure for introducing conveyed fluid into a user, the delivery structure being fluidly connected to the access structure, the conveyor support and the reservoir support being capable of diverging relative motion from a first relative position to a second relative position and converging relative motion from the second relative position to the first relative position, B) a cap removably mountable onto the dispensing unit to cover at least a portion of the conveyor support, and C) a coupling mechanism configured to bring the conveyor support and the reservoir support from the first relative position to the second relative position in response to the cap being mounted onto the dispensing unit.

The fluid conveying structure may be a needle assembly as described in the above in accordance with the other aspects of the invention, in which case the delivery structure may comprise e.g. a subcutaneous injection needle or an IV infusion set. Alternatively, the fluid conveying structure may be adapted for oral application, in which case the delivery structure may comprise e.g. a mouthpiece for inhalation or for enteral fluid delivery.

The coupling mechanism may further be configured to bring the conveyor support and the reservoir support from the second relative position to the first relative position in response to the cap being dismounted from the dispensing unit.

In the present context the term "distal" refers to a portion or position close to the outlet end (e.g. the needle end or the needle receiving end) of the drug delivery device, and "proximal" refers to a portion or position opposite the outlet end of the drug delivery device. In accordance therewith the term "distally" refers to a direction from a proximal position towards a distal position, and the term "proximally", conversely, refers to a direction from a distal position towards a proximal position.
Also, in the present context the term "needle assembly" covers a needle assembly as known in the use of conventional injection pens, i.e. an assembly comprising a hollow needle tubing, a needle hub to which the hollow needle tubing is affixed, and a collar with means for attachment to a receiving part of the injection pen, as well as any other type of needle assembly comprising a hollow needle tubing, and a hollow needle tubing as a stand-alone element.
Furthermore, the term "variable volume reservoir" is to be understood as a reservoir structure comprising a chamber for holding a liquid substance, and an outlet portion, which chamber is reducible in volume to cause at least some of the liquid substance to be expelled through the outlet portion. One example of a variable volume reservoir is a cartridge having a generally cylindrical body sealed respectively by a penetrable septum and a displaceable piston. Such a cartridge is e.g. known from the diabetes care segment for containment of drugs such as insulin and glp-1. Another example is a flexible pouch with a penetrable section.
In the present specification, reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in, or inherent of, at least that one aspect or embodiment of the invention, but not necessarily in/of all aspects or embodiments of the invention. It is emphasized, however, that any combination of the various features, structures and/or characteristics described in relation to the invention is encompassed by the invention unless expressly stated herein or clearly contradicted by context. The invention is defined by the appended claims. The use of any and all examples, or exemplary language (e.g., such as, etc.), in the text is intended to merely illuminate the invention and does not pose a limitation on the scope of the same, unless otherwise claimed. Further, no language or wording in the specification should be construed as indicating any non-claimed element as essential to the practice of the invention.

### BRIEF DESCRIPTION OF THE DRAWINGS

In the following the invention will be further described with references to the drawings, wherein
Figs. 1 and 2 illustrate a relative motion between a reservoir, or a reservoir support, and a needle support carrying an injection needle, according to the present invention,
Fig. 3 is a perspective view of a drug delivery device according to a first embodiment of the invention,
Fig. 4 is a perspective view showing the interior of the cap,
Fig. 5a is a perspective view of the drug delivery device in an initial cap mounting stage,
Fig. 5b is a close up view of a section of Fig. 5a, detailing elements of the coupling mechanism,
Fig. 6 is a perspective partial section view of the coupling mechanism,
Figs. 7-9 are perspective views of the drug delivery device during different stages of cap mounting, showing the gradual withdrawal of the drug delivery needle from the reservoir interior,
Fig. 10 is an exploded perspective view of a distal portion of a drug delivery device according to a second embodiment of the invention,
Fig. 11 is a perspective view of the needle support,
Fig. 12a is a perspective view of the reservoir support in connection with the needle support,
Fig. 12b is an enlargement of the area Q2 in Fig. 12a,
Figs. 13-16 are perspective views of the reservoir support and the needle support during different stages of cap mounting,
Fig. 17 is a side view of the reservoir support and the needle support corresponding to Fig. 15,
Fig. 18 is a side view of the reservoir support and the needle support corresponding to Fig. 16,
Fig. 19 is a perspective view of a drug delivery device according to a third embodiment of the invention,
Fig. 20 is a partly exploded perspective view of the drug delivery device of Fig. 19,
Fig. 21 is a close up perspective view of an element of the coupling mechanism, and
Figs. 22 and 23 are perspective views of the drug delivery device during different stages of cap mounting.

In the figures like structures are mainly identified by like reference numerals.

### DESCRIPTION OF EXEMPLARY EMBODIMENTS

When in the following relative expressions, such as "upwards" and "downwards", "upper" and "lower", and "clockwise" and "counter-clockwise", are used these refer to the appended figures and not necessarily to an actual situation of use. The shown figures are schematic representations for which reason the configuration of the different structures as well as their relative dimensions are intended to serve illustrative purposes only.

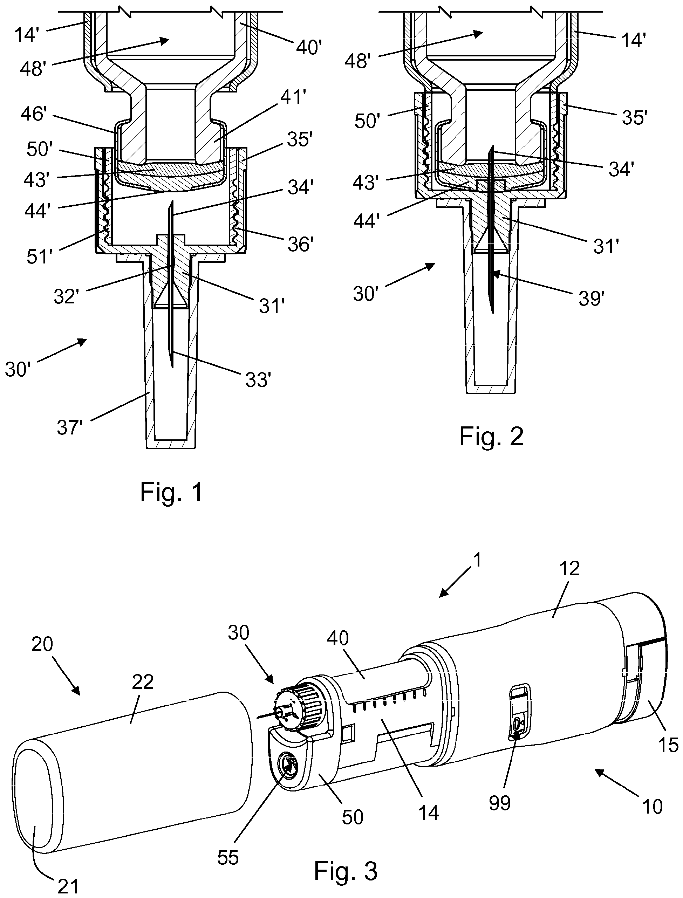

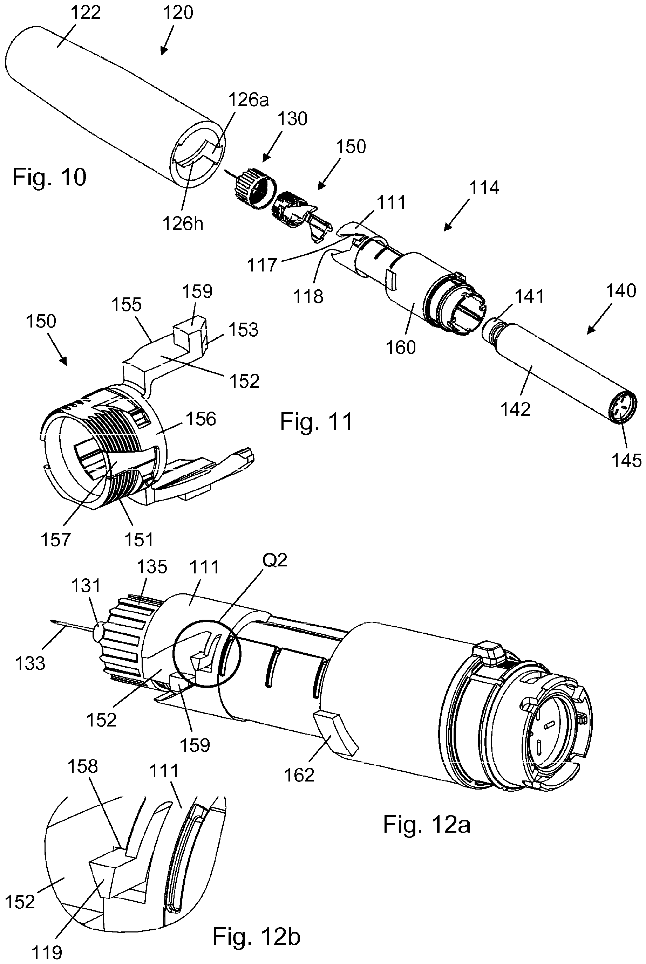

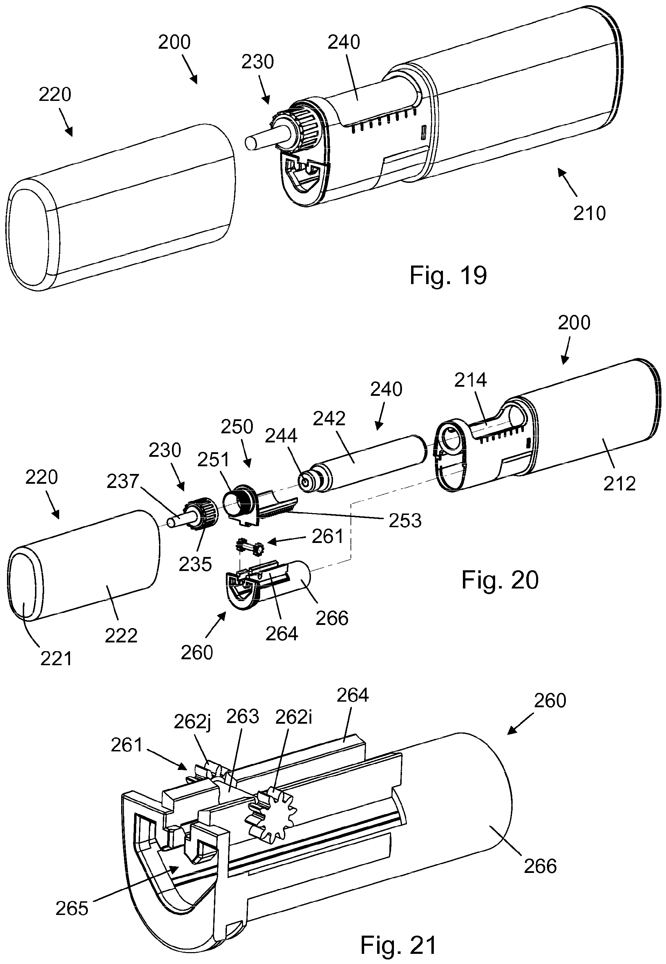

In the below three different manifestations of the present invention are described with reference to the figures, of which figs. 3-9 illustrate a first embodiment of the invention in the context of a dual axis dose setting and injection device, figs. 10-18 illustrate a second embodiment of the invention in the context of a conventional type single axis tubular injection pen, and figs. 19-23 illustrate a third embodiment of the invention in the context of a dual axis fixed dose delivery device.

The inventive concept underlying each of these different embodiments is an automatic induction of a diverging relative motion between a support structure for a drug delivery pathway, such as e.g. an injection needle, and a reservoir in response to a mounting of a removable cap onto a drug delivery unit to thereby interrupt a fluid connection between a supported drug delivery pathway and an interior of the reservoir. This will ensure that even if a user chooses to leave the drug delivery pathway on the support structure after a completed drug delivery procedure then when the cap is mounted on the drug delivery unit, e.g. for storage of the drug delivery device, the drug delivery pathway will be disconnected from the reservoir, whereby undesired effects such as drug leakage, clogging of the drug delivery pathway, and air entry into the reservoir interior are avoided.

Figs. 1 and 2 together sketch the basic movement pattern realised in every embodiment of the present invention. Fig. 1 shows a needle assembly 30' removably mounted on a needle holder 50' which is associated with a drug delivery unit of e.g. an injection device or an infusion pump. The needle assembly 30' comprises a needle hub 31' to which a needle cannula 32' is fixedly attached, e.g. by gluing. A generally cylindrical collar 35' extends from the needle hub 31' towards a drug containing cartridge 40', also being associated with the drug delivery unit. An inner surface portion of the collar 35' is provided with a thread 36' which is configured for secure engagement with a thread 51' on an outer surface portion of the needle holder 50'.

The needle cannula 32' extends through the needle hub 31' such that a back needle 34' projects from the needle hub 31' in a proximal direction and a front needle 33' projects from the needle hub 33' in a distal direction. The front needle 33' is adapted for insertion into a subcutaneous tissue of a person, and the back needle 34' is adapted for establishing fluid communication to a cartridge interior 48' holding the drug (not shown). In a pre-use state the front needle 33' is covered by a needle cap 37' to avoid premature exposure of the sharpened needle end.

The cartridge 40' extends upwards along a longitudinal axis and is retained in the axial and radial directions by a cartridge holder 14'. At its distal end portion the cartridge 40' is sealed by a penetrable septum comprising a primary septum layer 43', made of a material which is compatible with the drug in the cartridge interior 48', and a self-sealing secondary septum layer 44', e.g. made of isoprene. The septum is press-fitted to a flange section 41' of the cartridge 40' by a crimped metal seal cap 46'.

In Fig. 1 the back needle 34' is axially spaced apart from the distal end portion of the cartridge 40, and the needle cannula 32' and the cartridge interior 48' are thus fluidly unconnected.

Fig. 2 shows the needle assembly 30' in a position in which the back needle 34' has penetrated both the primary septum layer 43' and the secondary septum layer 44' and established fluid communication with the cartridge interior 48'. The drug is at this point able to flow through a lumen 39' in the needle cannula 32' and exit the sharpened end portion of the front needle 33'.

In accordance with the invention the needle holder 50' and the cartridge holder 14' are capable of undergoing relative axial motion between the relative position depicted in Fig. 1 and the relative position depicted in Fig. 2, and as will be explained in greater detail below such relative axial motion can be induced by the mounting, respectively dismounting, of a cap onto/from the drug delivery unit. From the fluidly unconnected state (Fig. 1) to the fluidly connected state (Fig. 2) the needle holder 50', carrying the needle assembly 30', and the cartridge holder 14', carrying the cartridge 40', undergo a converging relative axial motion, whereas from the fluidly connected state to the fluidly unconnected state the needle holder 50'/needle assembly 30' and the cartridge holder 14'/cartridge 40' undergo a diverging relative axial motion.

Fig. 3 is a perspective view of an injection device 1 according to a first embodiment of the invention. The injection device 1 comprises a drug delivery unit 10 and a protective cap 20. The drug delivery unit 10 comprises a housing 12 which extends along a general longitudinal axis and accommodates a drug expelling mechanism, a cartridge holder 14 which is axially fixed to the housing 12 and which carries a cartridge 40, and a needle holder 50 to which a needle assembly 30 is removably attached. The needle holder 50 has a bore 55 adapted for reception of a portion of the cap 20, as will be explained in greater detail below. An injection button 15 is provided at the proximal end portion of the housing 12 for activation of the drug expelling mechanism. The injection device 1 is capable of selective dose setting, and the housing 12 thus further accommodates a dose setting mechanism which comprises a scale drum having dose indicia applied to its exterior surface. A window 99 is provided in the housing 12 for inspection of the dose indicia, as conventionally known in the art of injection devices. The dose setting mechanism is user operable, but as the dose setting procedure is irrelevant to the invention further description thereof is omitted from the present text. The cap 20 comprises an axially extending circumferential side wall 22, and an end wall 21, and is adapted to be removably mounted onto the drug delivery unit 10 so as to cover and protect the needle holder 50, the cartridge holder 14, and the cartridge 40.

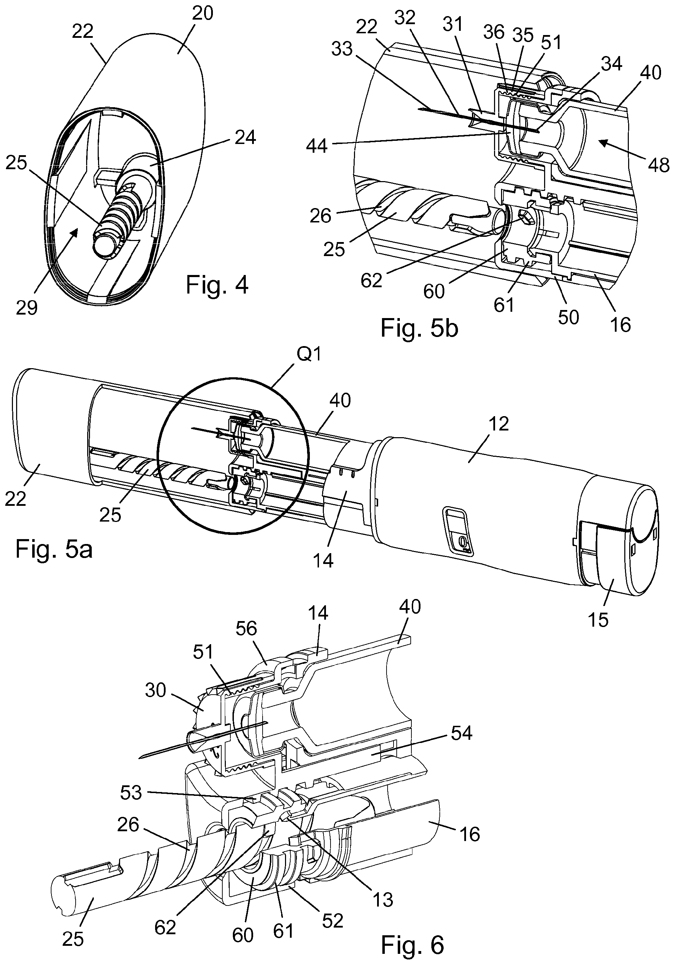

Fig. 4 shows the cap 20 from a proximal viewpoint. The end wall 21 and the side wall 22 together provide a hollow 29 in which a rod 25 extends in the proximal direction from a retainer 24 fixed to the end wall 21. The retainer 24 serves to axially and rotationally fix the rod 25 with respect to the end wall 21.

Fig. 5a shows the injection device 1 at the initiation of a mounting of the cap 20 onto the drug delivery unit 10. A portion of the side wall 22 has been cut out of the figure to show how the rod 25 is designed to align with the needle holder 50.

Fig. 5b is a close up view of the area Q1 in Fig. 5a, showing details of a coupling mechanism that serves to induce a relative axial motion between the needle holder 50 and the cartridge 40 in response to a relative axial motion between the cap 20 and the drug delivery unit 10 during mounting of the cap 20 onto, or dismounting of the cap 20 from, the drug delivery unit 10. A tube 16 is fixedly connected with the housing and extends axially parallel to the cartridge 40. A nut 60 is axially fixed to the tube 16 but freely rotatable in both directions about its centre axis. The nut 60 comprises an exterior thread 61 for engagement with the needle holder 50 and an interior protrusion 62 for engagement with a non-self-locking thread 26 on the rod 25. During the continued mounting of the cap 20 onto the drug delivery unit 10 the protrusion 62 will enter the thread 26 and travel at least a portion of it.

The needle assembly 30 comprises a needle cannula 32 fixedly retained in a needle hub 31 such that a front needle 33 extends in a distal direction from the needle hub 31 and a back needle 34 extends in a proximal direction from the needle hub 31. A collar 35 having an interior thread 36 extends from the needle hub 31 in the proximal direction. The thread 36 is in releasable engagement with a needle mount 51 and is thereby axially fixed on the needle holder 50. The back needle 34 extends through a secondary septum layer 44 and a primary septum layer 43, together constituting a self-sealing septum for the cartridge 40, and resides in a cartridge interior 48.

Fig. 6 is a perspective partial section view of the coupling mechanism during mounting of the cap 20 onto the drug delivery unit 10 (the cap 20 is omitted from the figure and portions of the nut 60 and the tube 16 have been removed for the sake of clarity). The cap 20 and the housing 12 have undergone a relative converging axial motion, whereby the rod 25 has entered through the bore 55 and the nut 60 to reach into the tube 16. The relative motion between the cap 20 and the housing 12 has been purely axial, and since the rod 25 is axially and rotationally fixed with respect to the cap 20 the interaction between the non-self-locking thread 26 and the protrusion 62 has caused the nut 60 to rotate clockwise about a catch portion 13 of the tube 16.

The needle holder 50 comprises a lower portion 52 configured to contain the nut 60, a middle portion 54 carrying a thread segment 53, and an upper portion 56 comprising the needle mount 51. The thread segment 53 mates with the exterior thread 61 such that a clockwise rotation of the nut 60 causes a distal movement of the needle holder 50 relative to the cartridge holder 14. Such distal movement of the needle holder 50 is accompanied by a like distal movement of the needle assembly 30 due to the threaded engagement between the two.

A relative diverging axial motion between the cap 20 and the housing 12 introduced during dismounting of the cap 20 from the drug delivery unit 10 will cause a retraction of the rod 25 through the nut 60 and the bore 55 and thereby a counter-clockwise rotation of the nut 60 relative to the tube 16 as the protrusion 62 travels backward in the thread 26. The engagement between the exterior thread 61 and the thread segment 53 causes a resulting proximal movement of the needle holder 50 relative to the cartridge holder 14.

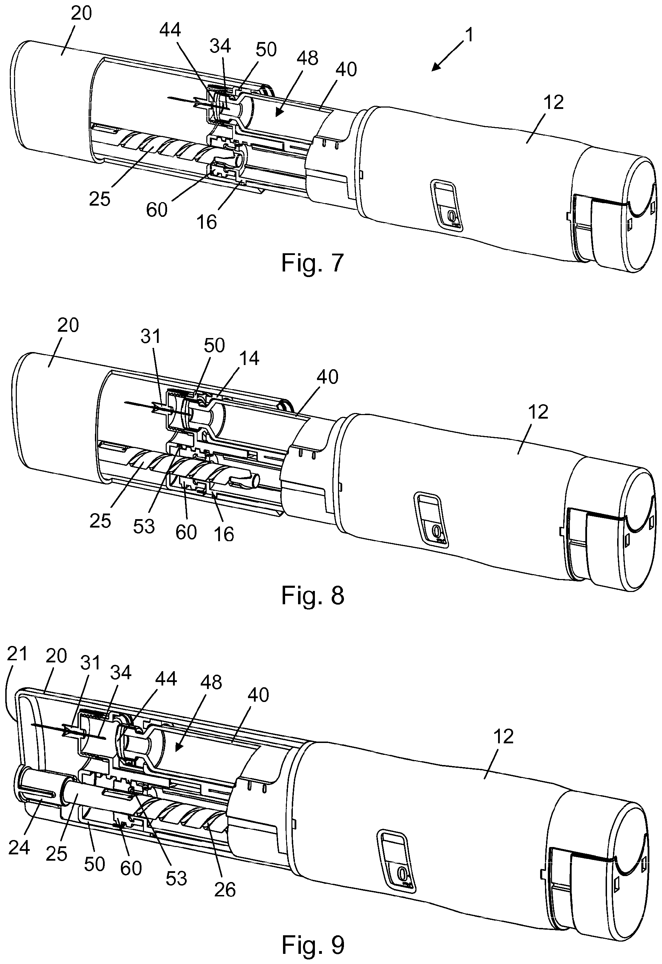

Figs. 7-9 show the injection device 1 at different stages during a mounting of the cap 20 onto the drug delivery unit 10. In Fig. 7 the free end portion of the rod 25 is situated within the nut 60, and the protrusion 62 is about to travel the thread 26. The back needle 34 still resides in the cartridge interior 48, i.e. fluid communication between the front needle 33 and the cartridge interior 48 is maintained. In Fig. 8 the cap 20 has been pushed further towards the housing 12, whereby the rod 25 has entered into the tube 16 and the protrusion 62 has travelled a portion of the thread 26. In accordance with what is explained in the above this has led to a clockwise rotation of the nut 60 and a distal displacement of the needle holder 50 and the needle hub 31 relative to the cartridge holder 14 and the cartridge 40. Only a small portion of the back needle 34 is still positioned in the cartridge interior 48. In Fig. 9 the cap 20 is fully mounted on the drug delivery unit 10 and the relative axial motion between the rod 25 and the nut 60 from the state of the injection device 1 shown in Fig. 8 to that of Fig. 9 has caused a further distal movement of the needle holder 50 relative to the cartridge holder 14, whereby the back needle 34 has been pulled out of the cartridge 40. The self-sealing secondary septum layer 44 has subsequently sealed the cartridge 40 and so the needle assembly is fluidly disconnected from the cartridge interior 48.

A cap mounting procedure is conventionally executed following each dose delivery, so, notably, no additional operating steps are introduced by the invention in order to achieve the automatic separation of the needle cannula 32 from the cartridge 40. Thereby, if a user decides to use the needle assembly 30 for more than one injection (s)he does not have to worry about whether the needle cannula 32 has become clogged or air has entered the cartridge interior 48 between the injections.

At the time of the following injection the user dismounts the cap 20 from the drug delivery unit 10, also a conventional action for users of an injection device, whereby the rod 25 moves distally relative to the nut 60, and the protrusion 62 is forced to travel the thread 26 in the opposite direction, causing the nut 60 to rotate counter-clockwise and the needle holder 50 to move proximally towards the cartridge holder 14. During this movement of the needle holder 50 the back needle 34 penetrates both the secondary septum layer 44 and the primary septum layer 43, and so when the cap 20 is completely dismounted from the drug delivery unit 10 the back needle 34 again resides in the cartridge interior 48, and the injection device 1 is ready for dose setting and injection.

Fig. 10 is an exploded perspective view of a distal portion of an injection device according to a second embodiment of the invention. The distal portion is composed of a protective cap 120 comprising a cap wall 122, which is closed at one end and open at an opposite end, a needle assembly 130, a needle holder 150 adapted to releasably retain the needle assembly 130, a cartridge holder 114, and a cartridge 140. The cartridge holder 114 is adapted for axial fixation to a tubular housing (not shown), e.g. in a manner conventionally known in the art of injection pens which comprises a proximal end portion of the cartridge holder 114 being retained by a distal end portion of the housing. The housing accommodates a drug expelling mechanism and constitutes together with the cartridge holder 114, the cartridge 140, and the needle holder 150 a drug delivery unit. The cartridge 140 is adapted for axial fixation in the cartridge holder 114 when the cartridge holder 114 is fixed to the housing.

The cartridge 140 comprises a cylindrical wall 142 which is sealed proximally by a displaceable piston 145. At its distal end the cartridge 140 has a flange section 141 which is closed by a self-sealing penetrable septum (not visible). When the cartridge 140 is fixed to the housing via the cartridge holder 114 the drug expelling mechanism is capable of exerting a driving force on the piston 145 via a piston rod (not shown).

The cartridge holder 114 comprises a radially expanded rear section 160 and a front section 111 for interaction with the needle holder 150. In particular, the front section 111 is formed with two carve outs (only one is visible) having respective opposing skewed faces 117, 118 for sliding reception of a proximal portion of the needle holder 150, as will be explained in the below.

Interior portions of the cap wall 22 are provided with grooves for reception and guiding of various protruding structures, the purpose of which grooves will be clear from the below. In Fig. 10 an axial groove section 126a and a helical groove section 126h at the open end portion of the cap wall 122 are visible. Diametrically opposite these groove sections a similar pair of groove sections is provided.

Fig. 11 is a perspective view detailing the needle holder 150. The needle holder 150 comprises a cylindrical distal portion 156, having thread sections 151 interrupted by bayonet tracks 157, providing means for reception of the needle assembly 130 in either a screw-on or a slide-and-twist fashion, and two proximally extending legs 152, each leg 152 having a skewed face 155 for sliding interaction with one of the skewed faces 117, and another skewed face 153 for sliding interaction with one of the skewed faces 118. Each leg 152 also has a radial protrusion 159.

Fig. 12a is a perspective view of the needle assembly 130, the needle holder 150, the cartridge holder 114, and the cartridge 140 in an assembled state. The needle assembly 130 comprises a collar 135 configured for releasable attachment to the distal portion 156 of the needle holder 150 by engagement with the bayonet tracks 157, and a needle hub 131 carrying a needle cannula in such a way that a front needle 133 extends distally therefrom and a back needle (not visible) extends proximally therefrom. In the assembled state shown in Fig. 12a a portion of the back needle resides in the interior of the cartridge 140. The rear section 160 of the cartridge holder 114 comprises two diametrically opposite radial protrusions 162 (only one is visible).

Fig. 12b is an enlarged view of the area Q2 of Fig. 12a, detailing a rotational lock between one of the legs 152 of the needle holder 150 and the front section 111 of the cartridge holder 114. The front section 111 includes an axially flexible finger 119 which is structured to interact with an edge 158 on the leg 152 to prevent relative rotation in one direction between the needle holder 150 and the cartridge holder 114 when the needle holder 150 and the cartridge holder 114 are in the depicted relative axial position. This rotational lock prevents separation of the needle holder 150 from the cartridge holder 114 when the cap 120 is not mounted on the drug delivery unit and enables easy attachment of a needle assembly to the needle holder 150 by a motion which involves rotation of the needle assembly.

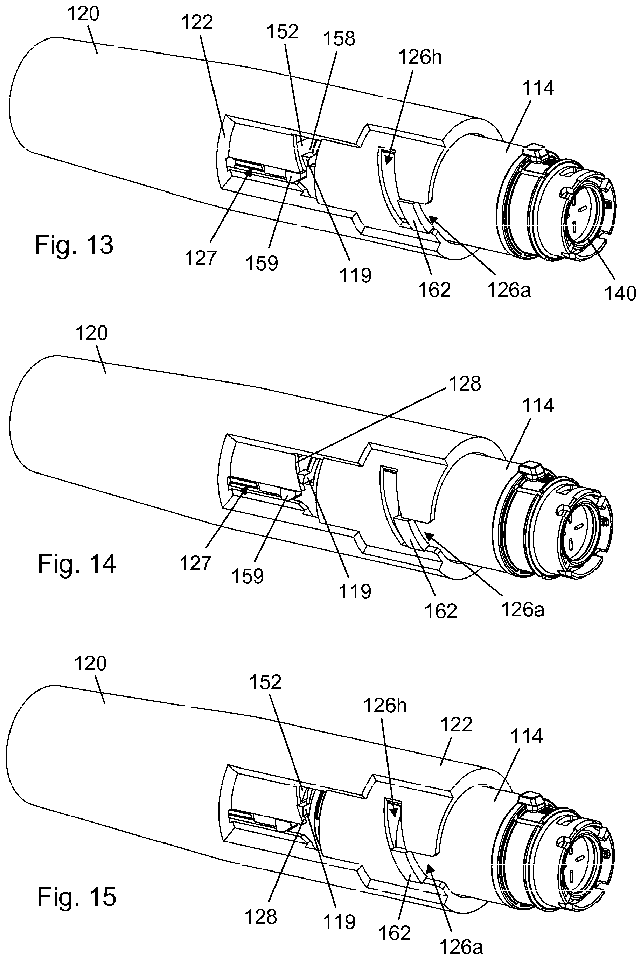

Figs. 13-16 show the needle holder 150 and the cartridge holder 114 in different states during mounting of the cap 120 onto the drug delivery unit. Different portions of the cap wall 22 have been removed to allow inspection of a coupling mechanism which serves to induce a relative axial motion between the needle holder 150 and the cartridge holder 114 in response to an axially converging motion between the cap 120 and the housing.

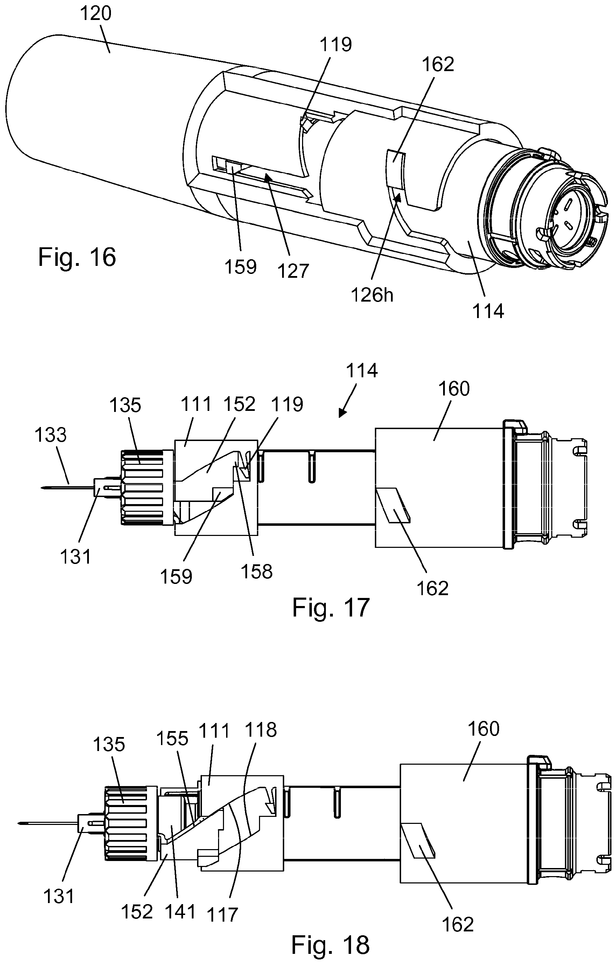

The cap 120 is mounted in a two-step procedure which involves firstly a pure linear axial relative motion and secondly a helical relative motion with respect to the housing and the cartridge holder 114. Figs. 13-15 illustrate internal movements and interactions during the linear part of the mounting, while Fig. 16 illustrates internal movements during the helical part of the mounting.

As can be seen from these figures the cap 120 has, apart from the axial groove section 126a and the helical groove section 126h, an additional axial groove 127 in an interior portion of the cap wall 122, which axial groove 127 is closer to the closed end of the cap wall 122 than the axial groove section 126a and is further of narrower width than the axial groove section 126a. A similar axial groove (not visible) is provided in the diametrically opposite portion of the cap wall 122.

In the following the internal interactions are described based on the single entities seen in the figures, instead of on the pair of entities actually present. It is, however, understood that these interactions are mirrored by the corresponding non-visible entities. When the cap 120 is slid linearly over the cartridge holder 114 the protrusion 162 is received in the axial groove section 126a and travels the axial groove section 126a gradually to the end thereof. Further, the protrusion 159 is received in the axial groove 127 and begins to travel the axial groove 127 towards the closed end of the cap wall 122.

As the protrusion 162 approaches the end of the axial groove section 126a a free surface 128 contacts the flexible finger 119 and causes the flexible finger 119 to deflect (Fig. 14). When the protrusion 162 reaches the end of the axial groove section 126a the free surface 128 has deflected the flexible finger 119 to such a degree that the needle holder 150 is no longer prevented from undergoing relative rotation with respect to the cartridge holder 114 (Fig. 15).

When the cap 120 and the cartridge holder 114 subsequently undergo relative rotation (Fig. 16) the protrusion 162 travels the helical groove section 126h, the flexible finger 119 slides along the free surface 128, and the protrusion 159 travels further distally in the axial groove 127, leading to an axial displacement of the needle holder 150 relative to the cartridge holder 114. Figs. 17 and 18 show the relative positions of the cartridge holder 114 and the needle holder 150 corresponding to the states shown in Figs. 15 and 16, respectively. In Fig. 17 the flexible finger 119 is deflected and has been forced out of engagement with the edge 158, enabling relative rotation between the front section 111 and the leg 152. As the cartridge holder 114 undergoes relative rotation with respect to the cap 120, and the protrusion 162 travels the helical groove section 126h the axial groove 127 prevents rotation of the needle holder 150 relative to the cap 120 due to the interaction with the protrusion 159. This causes the skewed faces 155, 153 to slide along the respective skewed faces 117, 118, whereby the needle holder 150 is urged away from the cartridge holder 114, and the back needle is retracted from the interior of the cartridge 140. Fig. 18 thus shows the position of the needle holder 150 relative to the cartridge holder 114 when the cap 120 is completely mounted on the drug delivery unit.

When the cap 120 is dismounted from the drug delivery unit before the next injection it firstly undergoes relative rotation with respect to the cartridge holder 114 for alignment of the protrusion 162 with the axial groove section 126a. This relative rotational motion causes the skewed faces 155, 153 to slide back along the respective skewed faces 117, 118, whereby the needle holder 150 is urged towards the cartridge holder 114, as the protrusion 159 slides proximally in the axial groove 127, and the back needle is re-inserted into the interior of the cartridge 140 through the self-sealing penetrable septum. Hence, when the cap 120 is subsequently completely removed from the drug delivery unit, by a linear relative motion that causes the protrusion 162 to slide out of the axial groove section 126a, fluid communication is already established between the needle cannula and the interior of the cartridge 140, and the drug delivery unit is ready for insertion of the front needle 133 into the skin of the user.

Fig. 19 is a perspective view of an injection device 200 according to a third embodiment of the invention. The design of the injection device 200 resembles the design of the injection device 1 according to the first embodiment of the invention. However, whereas the injection device 1 offers variable dose setting the injection device 200 is a fixed dose delivery device which expels the same dose at each activation of its injection mechanism (not visible). The injection device 200 comprises a drug delivery unit 210 and a protective cap 220 removably mountable there onto. The drug delivery unit 210 carries a drug containing cartridge 240 and an exchangeable needle assembly 230.

Fig. 20 shows a partly exploded view of the injection device 200. The drug delivery unit 210 comprises a casing or housing 212 and a cartridge holder 214 axially and rotationally fixed to the housing 212. The cartridge holder 214 supports a drug containing cartridge 240 having a cylindrical side wall 242, of e.g. glass or plastic, and being closed at its distal end by a self-sealing septum 44. An axially displaceable needle holder 250 has a needle mount 251 for reception of a collar 235 of a needle assembly 230, and an axially extending rack 253 on the underside of a semi-cylindrical structure. The needle assembly 230 is of the type shown in Figs. 1 and 2, i.e. it comprises a needle cannula (not visible) fixedly attached to a needle hub (also not visible). The needle cannula comprises a front needle (not visible) adapted for providing subcutaneous entry, and a back needle (also not visible) adapted for establishing fluid communication with an interior of the cartridge 240 through the septum 44. A needle cap 237 is provided for protection of the front needle. In the cartridge holder 214 below the cartridge 240 a converter 260 is arranged. The converter 260 has a generally cylindrical base 266 on the top of which two axially extending circumferentially spaced apart rails 264 are provided, each of the rails 264 having a carve out for supporting a twin gear wheel 261. The cap 220 comprises a longitudinally extending circumferential side wall 222 which is closed at one end by a transversal end wall 221.

Fig. 21 is a perspective view detailing the converter 260. It is seen that the twin gear wheel 261 comprises a pair of gear wheels 262i, 262j connected by a shaft 263 which is rotatably retained in the carve outs of the rails 264. The base 266 is hollow and has a front opening 265 for reception of an interior portion of the cap 220, as explained in the below. The gear wheels 262i, 262j are configured for engagement with the rack 253 on the needle holder 250.

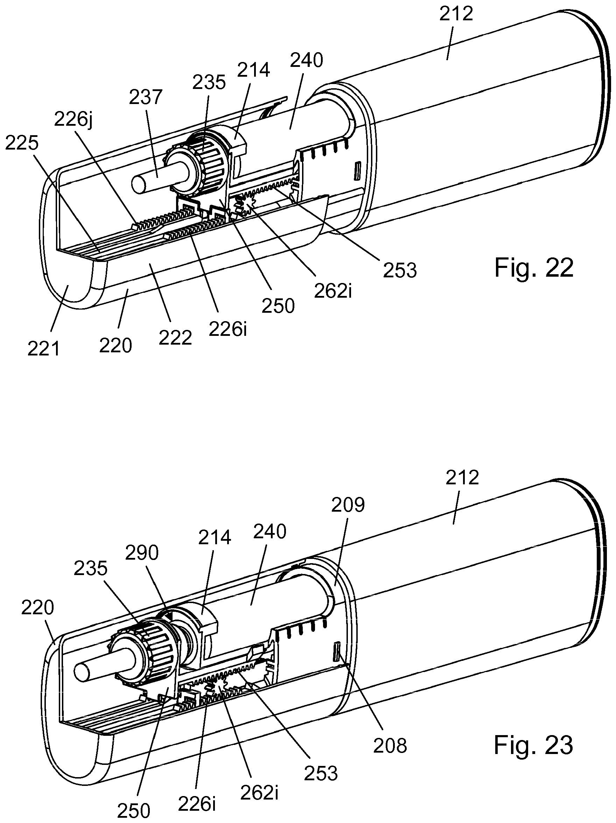

Figs. 22 and 23 show the injection device 200 at two different stages during mounting of the cap 220 onto the drug delivery unit 210. In both figures a portion of the side wall 222 has been removed to provide an overview of a coupling mechanism serving to displace the needle holder 250 relative to the cartridge holder 214 when the cap 220 moves relative to the housing 212.

An axial shelf 225 is provided in the interior of the cap 220. The shelf 225 holds a pair of racks 226i, 226j adapted for engagement with the respective gear wheels 262i, 262j. In Fig. 22 the cap 220 is only partly mounted on the drug delivery unit 210, and the gear wheels 262i, 262j are just about to enter into engagement with the racks 226i, 226j. The needle holder 250 and the cartridge holder 214 are next to one another such that a portion of the back needle of the attached needle assembly 230 resides in the interior of the cartridge 240.

In Fig. 23 the cap 220 is completely mounted onto the drug delivery unit 210. The open end of the cap 220 is received by a cap receiving portion 209 and releasably retained thereon via a snap-fitting engagement with a couple of protrusions 208 (only one is visible) on the cartridge holder 214. During the axially converging relative motion between the cap 220 and the housing 212 from the state of the injection device 200 shown in Fig. 22 to that shown in Fig. 23 the racks 226i, 226j have forced the gear wheels 262i, 262j to rotate counter-clockwise, whereby the rack 253 has been forced distally and, accordingly, the needle holder 250 and the cartridge holder 214 have undergone a diverging relative axial motion. The racks 226i, 226j, the gear wheels 262i, 262j, and the rack 253 thus together act as a double rack-and-pinion drive during mounting of the cap 220 onto, and dismounting of the cap 220 from, the drug delivery unit 210. Hence, when the cap 220 is completely mounted on the drug delivery unit 210 a space 290 is provided between the needle holder 250 and the cartridge holder 214, and the back needle is pulled out of the septum 244, preventing any air from entering into the interior of the cartridge 240 and any drug from leaking out of the cartridge 240 through the attached needle cannula.

When the cap 220 is subsequently dismounted from the drug delivery unit 210 in order to perform the next injection the relative motions are reversed, and the double rack-and-pinion drive converts the diverging motion between the cap 220 and the housing 212 to a converging motion between the needle holder 250 and the cartridge holder 214, whereby the back needle again penetrates the septum 244 and fluid connection to the interior of the cartridge 240 is automatically established.

## Claims

1. A drug delivery device (1, 200) comprising:
- a drug delivery unit (10, 210) comprising:
∘ a housing (12, 212) extending along a general axis,
∘ a variable volume reservoir (40', 40, 140, 240), and
∘ a needle support (50', 50, 150, 250) for receiving a needle assembly (30', 30, 130, 230), the needle support (50', 50, 150, 250) and the variable volume reservoir (40', 40, 140, 240) being capable of diverging relative axial motion from a first relative position to a second relative position and converging relative axial motion from the second relative position to the first relative position,
- a cap (20, 120, 220) removably mountable onto the drug delivery unit (10, 210) to cover at least a portion of the needle support (50', 50, 150, 250), and
- a coupling mechanism configured to bring the needle support (50', 50, 150, 250) and the variable volume reservoir (40', 40, 140, 240) from the first relative position to the second relative position in response to the cap (20, 120, 220) being mounted onto the drug delivery unit (10, 210).

2. A drug delivery device according to claim 1, wherein the coupling mechanism is further configured to bring the needle support (50', 50, 150, 250) and the variable volume reservoir (40', 40, 140, 240) from the second relative position to the first relative position in response to the cap (20, 120, 220) being dismounted from the drug delivery unit (10, 210).

3. A drug delivery device according to claim 1 or 2, wherein the first relative position is a predetermined relative position.

4. A drug delivery device according to any of claims 1 - 3, wherein the second relative position is a predetermined relative position.

5. A drug delivery device according to any of the preceding claims, wherein the variable volume reservoir (40', 40, 140, 240) is axially fixed with respect to the housing (12, 212), and wherein the coupling mechanism is configured to displace the needle support (50', 50, 150, 250) distally in response to a converging relative axial motion between the cap (20, 120, 220) and the housing (12, 212) during mounting of the cap (20, 120, 220) onto the drug delivery unit (10, 210).

6. A drug delivery device according to claim 5, wherein the coupling mechanism is further configured to displace the needle support (50', 50, 150, 250) proximally in response to a diverging relative axial motion between the cap (20, 120, 220) and the housing (12, 212) during dismounting of the cap (20, 120, 220) from the drug delivery unit (10, 210).

7. A drug delivery device according to any of the preceding claims, wherein the needle support (50', 50, 150, 250) is configured to releasably retain the needle assembly (30', 30, 130, 230).

8. A drug delivery device according to any of the preceding claims, further comprising the needle assembly (30', 30, 130, 230) being attached to the needle support (50', 50, 150, 250),
wherein the variable volume reservoir (40', 40, 140, 240) comprises a reservoir interior (48' 48) and a penetrable self-sealing septum (44', 44, 244),
wherein the needle assembly (30', 30, 130, 230) comprises a hollow access structure (34', 34) configured to penetrate the self-sealing septum (44', 44, 244), and
wherein in the first relative position the hollow access structure (34', 34) is fluidly connected with the reservoir interior (48', 48) through the self-sealing septum (44', 44, 244), and in the second relative position the hollow access structure (34', 34) is fluidly disconnected from the reservoir interior (48', 48).

9. A drug delivery device according to any of the preceding claims, wherein the coupling mechanism comprises a first interface structure (25, 127, 225) arranged in the cap (20, 120, 220), a second interface structure (53, 159, 253) arranged on the needle support (50', 50, 150, 250), and a third interface structure (60, 160, 260), each of the first interface structure (25, 127, 225), the second interface structure (53, 159, 253), and the third interface structure (60, 160, 260) being configured for interaction with at least one of the other of the first interface structure (25, 127, 225), the second interface structure (53, 159, 253), and the third interface structure (60, 160, 260) during mounting of the cap (20, 120, 220) onto, and dismounting of the cap (20, 120, 220) from, the drug delivery unit (10, 210).

10. A drug delivery device according to claim 9, wherein the needle support (50) comprises a first needle support portion (56) adapted to hold the needle assembly (30) and a second needle support portion (52) adapted to receive the first interface structure (25).

11. A drug delivery device according to claim 10, wherein the first needle support portion (56) and the second needle support portion (52) extend along the general axis and are arranged eccentrically.

12. A drug delivery device according to claim 11, wherein the second needle support portion (52) comprises the second interface structure (53), and
wherein the third interface structure (60) is axially fixed with respect to the housing and comprises an exterior portion configured for threaded engagement with the second interface structure (53), and an interior portion configured for threaded engagement with the first interface structure (25), such that an axial movement of the first interface structure (25) relative to the third interface structure (60) causes an opposite axial movement of the second needle support portion (52) relative to the third interface structure (60).

13. A drug delivery device according to claim 12, wherein the cap (20) comprises a longitudinally extending side wall (22) and a transversal end wall (21), together providing a hollow interior (29),
wherein the first interface structure (25) comprises a rod being translationally and rotationally fixed to the transversal end wall (21) and extending longitudinally in the hollow interior (29),
wherein the third interface structure (60) is a nut element being rotatably accommodated in the second needle support portion (52),
wherein one of the rod and the nut element comprises a first thread, and the other of the rod and the nut element comprises a first protruding structure configured to engage with the first thread, and
wherein one of the nut element and the second interface structure (53) comprises a second thread, and the other of the nut element and the second interface structure (53) comprises a second protruding structure configured to engage with the second thread.

14. A drug delivery device according to claim 9, further comprising a reservoir support (114) for axially retaining the variable volume reservoir (140), the reservoir support (114) being configured for axial and rotational fixation with respect to the housing,
wherein the needle support (150) and the reservoir support (114) are capable of undergoing relative helical motion during mounting of the cap (120) onto the drug delivery unit,
wherein the mounting of the cap (120) onto the drug delivery unit involves a relative angular displacement between the cap (120) and the reservoir support (114), and
wherein the first interface structure (127) and the second interface structure (159) are configured to rotationally fix the needle support (150) with respect to the cap (120).

15. A drug delivery device (1, 200) comprising:
- a drug delivery unit (10, 210) comprising:
∘ a housing (12, 212) extending along a general axis,
∘ a reservoir support (14', 14, 114, 214) for holding a variable volume reservoir, and
∘ a needle support (50', 50, 150, 250) for receiving a needle assembly (30', 30, 130, 230), the needle support (50', 50, 150, 250) and the reservoir support (14', 14, 114, 214) being capable of diverging relative axial motion from a first relative position to a second relative position and converging relative axial motion from the second relative position to the first relative position,
- a cap (20, 120, 220) removably mountable onto the drug delivery unit (10, 210) to cover at least a portion of the needle support (50', 50, 150, 250), and
- a coupling mechanism configured to bring the needle support (50', 50, 150, 250) and the reservoir support (14', 14, 114, 214) from the first relative position to the second relative position in response to the cap (20, 120, 220) being mounted onto the drug delivery unit (10, 210).

## Patentansprüche

1. Medikamenten-Verabreichungsvorrichtung (1, 200), umfassend:
- eine Medikamenten-Verabreichungseinheit (10, 210), umfassend:
∘ ein Gehäuse (12, 212), das sich entlang einer allgemeinen Achse erstreckt,
∘ ein Reservoir (40', 40, 140, 240) mit variablem Volumen und
∘ eine Nadelstütze (50', 50, 150, 250) zur Aufnahme einer Nadelanordnung (30', 30, 130, 230), wobei die Nadelstütze (50', 50, 150, 250) und das Reservoir (40', 40, 140, 240) mit variablem Volumen zu divergierender axialer Relativbewegung aus einer ersten Relativposition in eine zweite Relativposition und zu konvergierender axialer Relativbewegung aus der zweiten Relativposition in die erste Relativposition in der Lage sind,
- eine Kappe (20, 120, 220), die abnehmbar auf die Medikamenten-Verabreichungseinheit (10, 210) montierbar ist, um mindestens einen Abschnitt der Nadelstütze (50', 50, 150, 250) abzudecken, und
- einen Koppelmechanismus, der dazu konfiguriert ist, die Nadelstütze (50', 50, 150, 250) und das Reservoir (40', 40, 140, 240) mit variablem Volumen als Reaktion auf das Montieren der Kappe (20, 120, 220) auf die Medikamenten-Verabreichungseinheit (10, 210) aus der ersten Relativposition in die zweite Relativposition zu bringen.

2. Medikamenten-Verabreichungsvorrichtung nach Anspruch 1, wobei der Koppelmechanismus ferner dazu konfiguriert ist, die Nadelstütze (50', 50, 150, 250) und das Reservoir (40', 40, 140, 240) mit variablem Volumen als Reaktion auf das Abmontieren der Kappe (20, 120, 220) von der Medikamenten-Verabreichungseinheit (10, 210) aus der zweiten Relativposition in die erste Relativposition zu bringen.

3. Medikamenten-Verabreichungsvorrichtung nach Anspruch 1 oder 2, wobei die erste Relativposition eine vorbestimmte Relativposition ist.

4. Medikamenten-Verabreichungsvorrichtung nach einem der Ansprüche 1 - 3, wobei die zweite Relativposition eine vorbestimmte Relativposition ist.

5. Medikamenten-Verabreichungsvorrichtung nach einem der vorhergehenden Ansprüche, wobei das Reservoir (40', 40, 140, 240) mit variablem Volumen bezüglich des Gehäuses (12, 212) axial festgelegt ist und wobei der Koppelmechanismus dazu konfiguriert ist, die Nadelstütze (50', 50, 150, 250) als Reaktion auf eine konvergierende axiale Relativbewegung zwischen der Kappe (20, 120, 220) und dem Gehäuse (12, 212) während des Montierens der Kappe (20, 120, 220) auf die Medikamenten-Verabreichungseinheit (10, 210) distal zu verschieben.

6. Medikamenten-Verabreichungsvorrichtung nach Anspruch 5, wobei der Koppelmechanismus ferner dazu konfiguriert ist, die Nadelstütze (50', 50, 150, 250) als Reaktion auf eine divergierende axiale Relativbewegung zwischen der Kappe (20, 120, 220) und dem Gehäuse (12, 212) während des Abmontierens der Kappe (20, 120, 220) von der Medikamenten-Verabreichungseinheit (10, 210) proximal zu verschieben.

7. Medikamenten-Verabreichungsvorrichtung nach einem der vorhergehenden Ansprüche, wobei die Nadelstütze (50', 50, 150, 250) dazu konfiguriert ist, die Nadelanordnung (30', 30, 130, 230) freigebbar zu halten.

8. Medikamenten-Verabreichungsvorrichtung nach einem der vorhergehenden Ansprüche, ferner umfassend die Nadelanordnung (30', 30, 130, 230), die an der Nadelstütze (50', 50, 150, 250) angebracht ist,
wobei das Reservoir (40', 40, 140, 240) mit variablem Volumen ein Reservoirinneres (48', 48) und ein durchstechbares selbstabdichtendes Septum (44', 44, 244) umfasst,
wobei die Nadelanordnung (30', 30, 130, 230) eine hohle Zugriffsstruktur (34', 34) umfasst, die dazu konfiguriert ist, das selbstabdichtende Septum (44', 44, 244) zu durchstechen, und
wobei die hohle Zugriffsstruktur (34', 34) in der ersten Relativposition durch das selbstabdichtende Septum (44', 44, 244) fluidisch mit dem Reservoirinneren (48', 48) verbunden ist und die hohle Zugriffsstruktur (34', 34) in der zweiten Relativposition fluidisch vom Reservoirinneren (48', 48) getrennt ist.

9. Medikamenten-Verabreichungsvorrichtung nach einem der vorhergehenden Ansprüche, wobei der Koppelmechanismus eine in der Kappe (20, 120, 220) angeordnete erste Schnittstellenstruktur (25, 127, 225), eine an der Nadelstütze (50', 50, 150, 250) angeordnete zweite Schnittstellenstruktur (53, 159, 253) und eine dritte Schnittstellenstruktur (60, 160, 260) umfasst, wobei die erste Schnittstellenstruktur (25, 127, 225), die zweite Schnittstellenstruktur (53, 159, 253) und die dritte Schnittstellenstruktur (60, 160, 260) jeweils zum Zusammenwirken mit mindestens einer anderen der ersten Schnittstellenstruktur (25, 127, 225), der zweiten Schnittstellenstruktur (53, 159, 253) und der dritten Schnittstellenstruktur (60, 160, 260) während des Montierens der Kappe (20, 120, 220) auf die Medikamenten-Verabreichungseinheit (10, 210) und des Abmontierens der Kappe (20, 120, 220) von der Medikamenten-Verabreichungseinheit (10, 210) konfiguriert sind.

10. Medikamenten-Verabreichungsvorrichtung nach Anspruch 9, wobei die Nadelstütze (50) einen zum Halten der Nadelanordnung (30) geeigneten ersten Nadelstützenabschnitt (56) und einen zur Aufnahme der ersten Schnittstellenstruktur (25) geeigneten zweiten Nadelstützenabschnitt (52) umfasst.

11. Medikamenten-Verabreichungsvorrichtung nach Anspruch 10, wobei sich der erste Nadelstützenabschnitt (56) und der zweite Nadelstützenabschnitt (52) entlang der allgemeinen Achse erstrecken und exzentrisch angeordnet sind.

12. Medikamenten-Verabreichungsvorrichtung nach Anspruch 11, wobei der zweite Nadelstützenabschnitt (52) die zweite Schnittstellenstruktur (53) umfasst und wobei die dritte Schnittstellenstruktur (60) bezüglich des Gehäuses axial festgelegt ist und einen zum Gewindeeingriff mit der zweiten Schnittstellenstruktur (53) konfigurierten äußeren Abschnitt und einen zum Gewindeeingriff mit der ersten Schnittstellenstruktur (25) konfigurierten inneren Abschnitt umfasst, so dass durch eine axiale Bewegung der ersten Schnittstellenstruktur (25) bezüglich der dritten Schnittstellenstruktur (60) eine entgegengesetzte axiale Bewegung des zweiten Nadelstützenabschnitts (52) bezüglich der dritten Schnittstellenstruktur (60) veranlasst wird.

13. Medikamenten-Verabreichungsvorrichtung nach Anspruch 12, wobei die Kappe (20) eine sich in Längsrichtung erstreckende Seitenwand (22) und eine Querendwand (21) umfasst, die zusammen ein hohles Inneres (29) bereitstellen, wobei die erste Schnittstellenstruktur (25) einen Stab umfasst, der translationsmäßig und drehungsmäßig an der Querendwand (21) festgelegt ist und sich in Längsrichtung in das hohle Innere (29) erstreckt,
wobei die dritte Schnittstellenstruktur (60) ein Mutterelement ist, das drehbar in dem zweiten Nadelstützenabschnitt (52) aufgenommen ist,
wobei der Stab oder das Mutterelement ein erstes Gewinde umfasst und das jeweils andere Element (Stab oder Mutterelement) eine erste vorragende Struktur umfasst, die dazu konfiguriert ist, mit dem ersten Gewinde in Eingriff zu kommen, und
wobei das Mutterelement oder die zweite Schnittstellenstruktur (53) ein zweites Gewinde umfasst und das jeweils andere Element (Mutterelement oder zweite Schnittstellenstruktur (53)) eine zweite vorragende Struktur umfasst, die dazu konfiguriert ist, mit dem zweiten Gewinde in Eingriff zu kommen.

14. Medikamenten-Verabreichungsvorrichtung nach Anspruch 9, ferner umfassend eine Reservoirstütze (114) zum axialen Halten des Reservoirs (140) mit variablem Volumen, wobei die Reservoirstütze (114) zur axialen und drehungsmäßigen Festlegung bezüglich des Gehäuses konfiguriert ist,
wobei die Nadelstütze (150) und die Reservoirstütze (114) in der Lage sind, während des Montierens der Kappe (120) auf die Medikamenten-Verabreichungseinheit eine spiralförmige Relativbewegung durchzuführen,
wobei es beim Montieren der Kappe (120) auf die Medikamenten-Verabreichungseinheit zu einer winkelförmigen Relativverschiebung zwischen der Kappe (120) und der Reservoirstütze (114) kommt und
wobei die erste Schnittstellenstruktur (127) und die zweite Schnittstellenstruktur (159) dazu konfiguriert sind, die Nadelstütze (150) bezüglich der Kappe (120) drehungsmäßig festzulegen.

15. Medikamenten-Verabreichungsvorrichtung (1, 200), umfassend:
- eine Medikamenten-Verabreichungseinheit (10, 210), umfassend:
∘ ein Gehäuse (12, 212), das sich entlang einer allgemeinen Achse erstreckt,
∘ eine Reservoirstütze (14', 14, 114, 214) zum Halten eines Reservoirs mit variablem Volumen und
∘ eine Nadelstütze (50', 50, 150, 250) zur Aufnahme einer Nadelanordnung (30', 30, 130, 230), wobei die Nadelstütze (50', 50, 150, 250) und die Reservoirstütze (14', 14, 114, 214) zu divergierender axialer Relativbewegung aus einer ersten Relativposition in eine zweite Relativposition und zu konvergierender axialer Relativbewegung aus der zweiten Relativposition in die erste Relativposition in der Lage sind,
- eine Kappe (20, 120, 220), die abnehmbar auf die Medikamenten-Verabreichungseinheit (10, 210) montierbar ist, um mindestens einen Abschnitt der Nadelstütze (50', 50, 150, 250) abzudecken, und
- einen Koppelmechanismus, der dazu konfiguriert ist, die Nadelstütze (50', 50, 150, 250) und die Reservoirstütze (14', 14, 114, 214) als Reaktion auf das Montieren der Kappe (20, 120, 220) auf die Medikamenten-Verabreichungseinheit (10, 210) aus der ersten Relativposition in die zweite Relativposition zu bringen.

## Revendications

1. Dispositif d'administration de médicament (1, 200), comprenant :
- une unité d'administration de médicament (10, 210), comprenant :
- un boîtier (12, 212) s'étendant le long d'un axe général,
- un réservoir à volume variable (40', 40, 140, 240), et
- un support d'aiguille (50', 50, 150, 250) pour recevoir un ensemble d'aiguille (30', 30, 130, 230), le support d'aiguille (50', 50, 150, 250) et le réservoir à volume variable (40', 40, 140, 240) étant capables d'effectuer un mouvement axial relatif de divergence d'une première position relative dans une deuxième position relative et d'effectuer un mouvement axial relatif de convergence de la deuxième position relative dans la première position relative,
- un capuchon (20, 120, 220) pouvant être monté de manière amovible sur l'unité d'administration de médicament pour couvrir au moins une partie du support d'aiguille (50', 50, 150, 250), et
- un mécanisme d'accouplement configuré pour amener le support d'aiguille (50', 50, 150, 250) et le réservoir à volume variable (40', 40, 140, 240) de la première position relative dans la deuxième position relative en réponse au montage du capuchon (20, 120, 220) sur l'unité de d'administration de médicament (10, 210).

2. Dispositif d'administration de médicament selon la revendication 1, dans lequel le mécanisme d'accouplement est en outre configuré pour amener le support d'aiguille (50', 50, 150, 250) et le réservoir à volume variable (40', 40, 140, 240) de la deuxième position relative dans la première position relative en réponse au démontage du capuchon (20, 120, 220) de l'unité d'administration de médicament (10, 210).

3. Dispositif d'administration de médicament selon la revendication 1 ou 2, dans lequel la première position relative est une position relative prédéterminée.

4. Dispositif d'administration de médicament selon l'une quelconque des revendications 1 à 3, dans lequel la deuxième position relative est une position relative prédéterminée.

5. Dispositif d'administration de médicament selon l'une quelconque des revendications précédentes, dans lequel le réservoir à volume variable (40', 40, 140, 240) est fixé axialement par rapport au boîtier (12, 212), et dans lequel le mécanisme d'accouplement est configuré pour déplacer le support d'aiguille (50', 50, 150, 250) distalement en réponse à un mouvement axial relatif de convergence entre le capuchon (20, 120, 220) et le boîtier (12, 212) lors du montage du capuchon (20, 120, 220) sur l'unité d'administration de médicament (10, 210).

6. Dispositif d'administration de médicament selon la revendication 5, dans lequel le mécanisme d'accouplement est en outre configuré pour déplacer le support d'aiguille (50', 50, 150, 250) proximalement en réponse à un mouvement axial relatif de divergence entre le capuchon (20, 120, 220) et le boîtier (12, 212) lors du démontage du capuchon (20, 120, 220) de l'unité d'administration de médicament (10, 210).

7. Dispositif d'administration de médicament selon l'une quelconque des revendications précédentes, dans lequel le support d'aiguille (50', 50, 150, 250) est configuré pour retenir de manière amovible l'ensemble d'aiguille (30', 30, 130, 230).

8. Dispositif d'administration de médicament selon l'une quelconque des revendications précédentes, comprenant en outre la fixation de l'ensemble d'aiguille (30', 30, 130, 230) au support d'aiguille (50', 50, 150, 250),
le réservoir à volume variable (40', 40, 140, 240) comprenant un intérieur de réservoir (48', 48) et un septum auto-scellant pouvant être percé (44', 44, 244),
l'ensemble d'aiguille (30', 30, 130, 230) comprenant une structure d'accès creuse (34', 34) configurée pour percer le septum auto-scellant (44', 44, 244), et
la structure d'accès creuse (34', 34), dans la première position relative, étant connectée fluidiquement à l'intérieur du réservoir (48', 48) par le biais du septum auto-scellant (44', 44, 244), et la structure d'accès creuse (34', 34), dans la deuxième position relative, étant déconnectée fluidiquement de l'intérieur du réservoir (48', 48).

9. Dispositif d'administration de médicament selon l'une quelconque des revendications précédentes, dans lequel le mécanisme d'accouplement comprend une première structure d'interface (25, 127, 225) disposée dans le capuchon (20, 120, 220), une deuxième structure d'interface (53, 159, 253) disposée sur le support d'aiguille (50', 50, 150, 250) et une troisième structure d'interface (60, 160, 260), chacune de la première structure d'interface (25, 127, 225), de la deuxième structure d'interface (53, 159, 253), et de la troisième structure d'interface (60, 160, 260) étant configurée de manière à interagir avec au moins l'une de l'autre de la première structure d'interface (25, 127, 225), de la deuxième structure d'interface (53, 159, 253), et de la troisième structure d'interface (60, 160, 260) lors du montage du capuchon (20, 120, 220) sur l'unité d'administration de médicament (10, 210) et du démontage du capuchon (20, 120, 220) de celle-ci.

10. Dispositif d'administration de médicament selon la revendication 9, dans lequel le support d'aiguille (50) comprend une première partie de support d'aiguille (56) prévue pour retenir l'ensemble d'aiguille (30) et une deuxième partie de support d'aiguille (52) prévue pour recevoir la première structure d'interface (25).

11. Dispositif d'administration de médicament selon la revendication 10, dans lequel la première partie de support d'aiguille (56) et la deuxième partie de support d'aiguille (52) s'étendent le long de l'axe général et sont agencées de manière excentrique.

12. Dispositif d'administration de médicament selon la revendication 11, dans lequel la deuxième partie de support d'aiguille (52) comprend la deuxième structure d'interface (53), et
dans lequel la troisième structure d'interface (60) est fixée axialement par rapport au boîtier et comprend une partie extérieure configurée pour s'engager par vissage avec la deuxième structure d'interface (53), et une partie intérieure configurée pour s'engager par vissage avec la première structure d'interface (25), de telle sorte qu'un mouvement axial de la première structure d'interface (25) par rapport à la troisième structure d'interface (60) provoque un mouvement axial opposé de la deuxième partie de support d'aiguille (52) par rapport à la troisième structure d'interface (60).

13. Dispositif d'administration de médicament selon la revendication 12, dans lequel le capuchon (20) comprend une paroi latérale s'étendant longitudinalement (22) et une paroi d'extrémité transversale (21), toutes deux constituant un intérieur creux (29), dans lequel la première structure d'interface (25) comprend une tige fixée en translation et en rotation à la paroi d'extrémité transversale (21), et s'étendant longitudinalement dans l'intérieur creux (29),
dans lequel la troisième structure d'interface (60) est un élément d'écrou reçu de manière rotative dans la deuxième partie de support d'aiguille (52),
dans lequel l'un de la tige et de l'élément d'écrou comprend un premier filetage et l'autre de la tige et de l'élément d'écrou comprend une première structure saillante configurée pour venir en prise avec le premier filetage, et
dans lequel l'un de l'élément d'écrou et de la deuxième structure d'interface (53) comprend un deuxième filetage et l'autre de l'élément d'écrou et de la deuxième structure d'interface (53) comprend une deuxième structure saillante configurée pour venir en prise avec le deuxième filetage.

14. Dispositif d'administration de médicament selon la revendication 9, comprenant en outre un support de réservoir (114) destiné à retenir axialement le réservoir à volume variable (140), le support de réservoir (114) étant configuré pour pouvoir être fixé axialement et en rotation par rapport au boîtier,
dans lequel le support d'aiguille (150) et le support de réservoir (114) sont aptes à subir un mouvement hélicoïdal relatif lors du montage du capuchon (120) sur l'unité d'administration de médicament,
dans lequel le montage du capuchon (120) sur l'unité d'administration de médicament implique un déplacement angulaire relatif entre le capuchon (120) et le support de réservoir (114), et
dans lequel la première structure d'interface (127) et la deuxième structure d'interface (159) sont configurées de manière à fixer en rotation le support d'aiguille (150) par rapport au capuchon (120).

15. Dispositif d'administration de médicament (1, 200), comprenant :
- une unité d'administration de médicament (10, 210), comprenant :
- un boîtier (12, 212) s'étendant le long d'un axe général,
- un support de réservoir (14', 14, 114, 214) pour retenir un réservoir à volume variable, et
- un support d'aiguille (50', 50, 150, 250) pour recevoir un ensemble d'aiguille (30', 30, 130, 230), le support d'aiguille (50', 50, 150, 250) et le support de réservoir (14', 14, 114, 214) étant capables d'effectuer un mouvement axial relatif de divergence d'une première position relative dans une deuxième position relative et d'effectuer un mouvement axial relatif de convergence de la deuxième position relative dans la première position relative,
- un capuchon (20, 120, 220) pouvant être monté de manière amovible sur l'unité d'administration de médicament (10, 210) pour couvrir au moins une partie du support d'aiguille (50', 50, 150, 250), et
- un mécanisme d'accouplement configuré pour amener le support d'aiguille (50', 50, 150, 250) et le support de réservoir (14', 14, 114, 214) de la première position relative dans la deuxième position relative en réponse au montage du capuchon (20, 120, 220) sur l'unité d'administration de médicament (10, 210).
